(19) Europäisches Patentamt
European Patent Office
Office européen des brevets

(11) **EP 4 123 302 A1**

(12) **EUROPEAN PATENT APPLICATION**
published in accordance with Art. 153(4) EPC

(43) Date of publication:
**25.01.2023 Bulletin 2023/04**

(21) Application number: **21771348.6**

(22) Date of filing: **02.03.2021**

(51) International Patent Classification (IPC):
***G01N 31/00*** (2006.01)    ***G01N 31/10*** (2006.01)

(52) Cooperative Patent Classification (CPC):
**G01N 31/00; G01N 31/10;** Y02A 50/20

(86) International application number:
**PCT/JP2021/007834**

(87) International publication number:
**WO 2021/187079 (23.09.2021 Gazette 2021/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.03.2020 JP 2020046718**

(71) Applicant: **SHIMADZU CORPORATION
Kyoto-shi, Kyoto 604-8511 (JP)**

(72) Inventors:
• **IHARADA, Takeshi
Kyoto-shi, Kyoto 604-8511 (JP)**
• **YAHATA, Masahito
Kyoto-shi, Kyoto 604-8511 (JP)**

(74) Representative: **Müller-Boré & Partner
Patentanwälte PartG mbB
Friedenheimer Brücke 21
80639 München (DE)**

(54) **TOTAL ORGANIC CARBON MEASUREMENT DEVICE AND TOTAL ORGANIC CARBON MEASUREMENT METHOD**

(57)    A sample heating unit 30 has a space in which an oxidation catalyst is arranged, and heats a sample arranged in the space. A carrier gas introduction unit 40 introduces inert gas containing water vapor as carrier gas into the sample heating unit 30. A detection unit 35 detects carbon dioxide generated by steam reforming reaction of organic carbon in a sample in the sample heating unit 30.

FIG.1

EP 4 123 302 A1

**Description**

TECHNICAL FIELD

The present invention relates to a total organic carbon measurement device and a total organic carbon measurement method.

BACKGROUND ART

In order to measure total organic carbon (TOC) contained in a sample, a total organic carbon measurement device is used. A sample may contain inorganic carbon (IC) in addition to total organic carbon. TOC and IC are collectively referred to as total carbon (TC).
As an example of a method of measuring TOC, a method of measuring TC and IC, and calculating a difference between them (TC - IC) as TOC is known (see, for example, Patent Document 1 below). TC is measured as carbon dioxide generated when a sample is burned in a combustion tube is detected by a detection unit. On the other hand, IC is measured as carbon dioxide generated when a sample is added with acid and aerated is detected by a detection unit.
Further, as another method for measuring TOC, a method of measuring TOC in which acid is added to a sample and then the sample is aerated so that IC is converted into carbon dioxide and the carbon dioxide is removed, and carbon dioxide generated when the sample from which the IC is removed is burned in a combustion tube is detected by a detection unit.
In either of the above two methods, carrier gas is introduced into a combustion tube, and a mixture of the carrier gas and a sample is burned in the combustion tube. As the carrier gas, high purity air or the like is used. The carrier gas has not only a function of sending carbon dioxide generated from a sample to a detection unit, but also a function as combustion supporting gas when the sample is burned in the combustion tube.

PRIOR ART DOCUMENT

PATENT DOCUMENT

Patent Document 1: Japanese Patent Laid-Open No. 2007-93209

SUMMARY OF THE INVENTION

PROBLEMS TO BE SOLVED BY THE INVENTION

In the conventional total organic carbon measurement device, since carrier gas also has a function as combustion supporting gas, it has been difficult to use inert gas as the carrier gas. That is, in a case where inert gas is used as carrier gas, carbon dioxide generated from a sample can be sent to a detection unit by the carrier gas, but it is difficult to efficiently burn the sample in a combustion tube.
The present invention has been made in view of the above circumstances, and an object of the present invention is to provide a total organic carbon measurement device and a total organic carbon measurement method in which inert gas can be used as carrier gas.

MEANS FOR SOLVING THE PROBLEMS

A first aspect of the present invention is a total organic carbon measurement device including a sample heating unit, a carrier gas introduction unit, and a detection unit. The sample heating unit has a space in which an oxidation catalyst is arranged, and heats a sample arranged in the space. The carrier gas introduction unit introduces inert gas containing water vapor as carrier gas into the sample heating unit. The detection unit detects carbon dioxide generated by steam reforming reaction of organic carbon in a sample in the sample heating unit.
A second aspect of the present invention is a total organic carbon measurement method including a carrier gas introduction step, a sample heating step, and a detection step. In the carrier gas introduction step, inert gas containing water vapor is introduced as carrier gas into a sample heating unit having a space in which an oxidation catalyst is arranged. In the sample heating step, a sample arranged in the space is heated in the sample heating unit, and organic carbon in the sample is caused to undergo steam reforming reaction, so that carbon dioxide is generated. In the detection step, the carbon dioxide that is generated is detected.

EFFECTS OF THE INVENTION

According to the present invention, carbon dioxide can be generated from total organic carbon in a sample by an action of water vapor contained in carrier gas, and therefore inert gas can be used as the carrier gas.

BRIEF DESCRIPTION OF THE DRAWINGS

FIG. 1 is a schematic view illustrating a configuration example of a total organic carbon measurement device.
FIG. 2 is a schematic cross-sectional view illustrating a configuration example of a humidifier.
FIG. 3 is a flowchart for explaining a method for measuring total organic carbon.

MODE FOR CARRYING OUT THE INVENTION

1. Overall configuration of total organic carbon measurement device

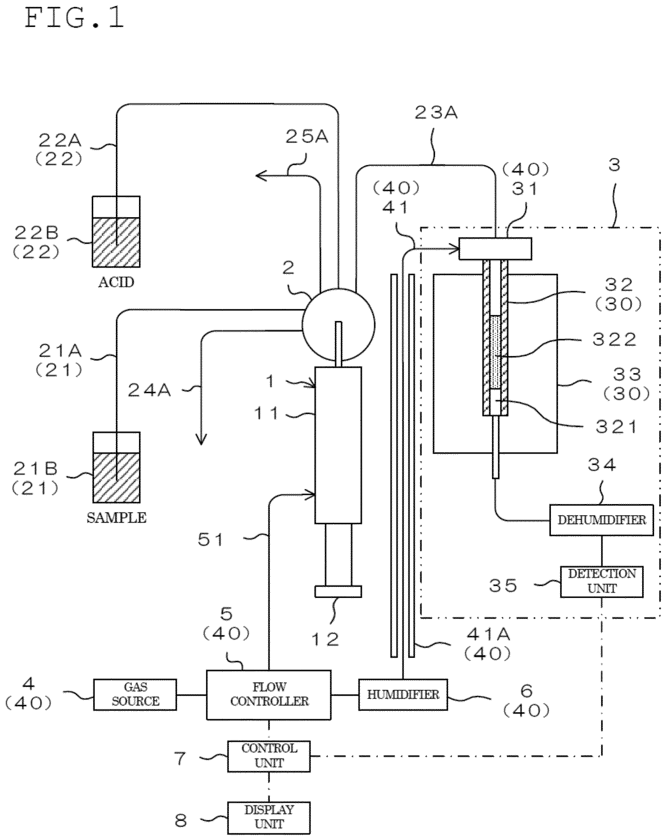

FIG. 1 is a schematic view illustrating a configuration example of a total organic carbon measurement device. The total organic carbon measurement device includes a syringe 1, a flow path switching unit 2, a total organic carbon measurement unit (TOC measurement unit) 3, a gas source 4, a flow controller 5, a humidifier 6, a control unit 7, a display unit 8, and the like.

The syringe 1 includes, for example, a cylindrical body 11 and a plunger 12. The plunger 12 is inserted into the cylindrical body 11, and liquid can be sucked into an internal space of the syringe 1 enclosed by an inner surface of the cylindrical body 11 and the plunger 12. Specifically, by displacing the plunger 12 with respect to the cylindrical body 11, suction operation of liquid into the syringe 1 and discharge operation of liquid from the syringe 1 are performed. The plunger 12 is displaced by driving of a drive unit (not illustrated) including, for example, a motor.

The syringe 1 is fluidly connected to the flow path switching unit 2. The flow path switching unit 2 includes, for example, a multi-port valve that allows optional communication of a plurality of ports. Pipes 21A, 22A, 23A, 24A, and 25A are fluidly connected to ports of the flow path switching unit 2, and any one of the pipes 21A, 22A, 23A, 24A, and 25A can communicate with the syringe 1 by switching of the flow path switching unit 2.

When suction operation by the syringe 1 is performed in a state where the pipe 21A communicates with the syringe 1, a sample to be analyzed is sucked into the syringe 1 from a sample storage unit 21B storing the sample. Further, in a case where suction operation by the syringe 1 is performed in a state where the pipe 22A communicates with the syringe 1, acid is sucked into the syringe 1 from an acid storage unit 22B storing the acid.

In this manner, a sample or acid can be selectively sucked into the syringe 1. As the acid, hydrochloric acid can be exemplified, but the acid is not limited to hydrochloric acid. The pipe 21A and the sample storage unit 21B constitute a sample supply unit 21 for supplying a sample. The pipe 22A and the acid storage unit 22B constitute an addition unit 22 for adding acid to a sample by introducing the acid into the syringe 1.

The pipe 23A fluidly connects the flow path switching unit 2 and the TOC measurement unit 3. In a case where discharge operation by the syringe 1 is performed in a state where the pipe 23A communicates with the syringe 1, liquid in the syringe 1 is supplied to the TOC measurement unit 3 via the pipe 23A. The pipe 24A communicates with the drain. The pipe 25A communicates with the atmosphere for degassing the syringe 1.

The TOC measurement unit 3 includes a sample injection mechanism 31, a reaction tube 32, an electric furnace 33, a dehumidifier 34, a detection unit 35, and the like. The pipe 23A communicates with the sample injection mechanism 31 in the TOC measurement unit 3. Therefore, liquid in the syringe 1 can be supplied from the pipe 23A to the sample injection mechanism 31 by discharge operation by the syringe 1.

In addition to the pipe 23A as a sample supply path, a pipe 41 as a carrier gas supply path is fluidly connected to the sample injection mechanism 31. The pipe 41 communicates the gas source 4 and the sample injection mechanism 31, and carrier gas supplied from the gas source 4 is introduced into the sample injection mechanism 31 via the pipe 41. Carrier gas supplied from the gas source 4 is, for example, inert gas such as high-purity nitrogen. However, the carrier gas is not limited to nitrogen, and may be another type of inert gas.

The flow controller 5 and the humidifier 6 are provided in the pipe 41. The humidifier 6 constitutes a water vapor generation unit that generates water vapor. By providing the humidifier 6 in the pipe 41 and allowing carrier gas to pass through the humidifier 6, water vapor generated in the humidifier 6 can be mixed with the carrier gas. In this manner, carrier gas mixed with water vapor can be introduced into the sample injection mechanism 31.

At least part of the pipe 41 is heated by a pipe heating unit 41A. The pipe heating unit 41A may heat at least part of the pipe 41 between the humidifier 6 and the sample injection mechanism 31. In this manner, water vapor in the carrier gas flowing in the pipe 41 can be heated by the pipe heating unit 41A. Temperature of the pipe 41 in a portion heated by the pipe heating unit 41A is adjusted to, for example, about 50°C. The pipe 41 is formed of, for example, metal such as stainless steel, and the pipe heating unit 41A is provided so as to be in contact with or close to the pipe 41 from the outside.

The pipe heating unit 41A can be composed of, for example, a sheath heater, but is not limited to a sheath heater. Further, it is also possible to omit the pipe heating unit 41A. That is, water vapor in carrier gas flowing in the pipe 41 does not need to be heated. In this case, temperature of water vapor may be 0°C or higher, and may be, for example, a temperature similar to room temperature.

The reaction tube 32 communicates with the sample injection mechanism 31, and a sample supplied from the syringe 1 to the sample injection mechanism 31 is injected into the reaction tube 32 together with carrier gas from the sample injection mechanism 31. The gas source 4, the flow controller 5, the humidifier 6, the sample injection mechanism 31, the pipe 41, and the pipe heating unit 41A constitute a carrier gas introduction unit 40 that introduces carrier gas into the reaction tube 32.

The reaction tube 32 is formed of, for example, quartz glass or ceramic, and an oxidation catalyst 322 is arranged in a space 321 formed inside. As the oxidation catalyst, for example, a platinum -supported alumina catalyst is used. The reaction tube 32 is externally heated by the electric furnace 33. The electric furnace 33 heats a sample arranged in the space 321 of the reaction tube 32 by heating the reaction tube 32 at a high temperature of about 680°C. The reaction tube 32 and the electric furnace 33 constitute a sample heating unit 30 for heating a sample. However, the sample heating unit 30 is not limited to one composed of the reaction tube 32 and the electric furnace 33, and may be composed of other members.

A sample injected into the reaction tube 32 from the sample injection mechanism 31 generates carbon dioxide as an organic substance contained in the sample is oxidized. An amount of carbon dioxide generated from a sample is an amount corresponding to an amount of an organic substance contained in the sample. Carbon dioxide generated in the reaction tube 32 is sent to the dehumidifier 34 together with carrier gas, dehumidified in the dehumidifier 34, and then guided to the detection unit 35.

The detection unit 35 can be composed of, for example, a non-dispersive infrared absorption type sensor (NDIR type sensor), but is not limited to a non-dispersive infrared absorption type sensor. The detection unit 35 detects carbon dioxide generated from a sample in the reaction tube 32. A carbon dioxide detection signal in the detection unit 35 is input to the control unit 7.

The control unit 7 includes, for example, a processor including a central processing unit (CPU). The control unit 7 calculates TOC contained in a sample by performing calculation based on a carbon dioxide detection signal input from the detection unit 35. Further, the control unit 7 controls operation of each unit such as the flow controller 5 provided in the total organic carbon measurement device.

The display unit 8 is composed of, for example, a liquid crystal display. Display on the display unit 8 is controlled by the control unit 7. The display unit 8 displays various types of information such as a measurement result of TOC, for example.

A pipe 51 communicating with the syringe 1 is connected to the flow controller 5 separately from the pipe 41 communicating with the sample injection mechanism 31. The flow controller 5 is a two-system flow controller capable of individually controlling a flow rate of carrier gas toward the sample injection mechanism 31 via the pipe 41 and a flow rate of carrier gas toward the syringe 1 via the pipe 51. The flow controller 5 constantly supplies carrier gas from the gas source 4 to the sample injection mechanism 31 via the pipe 41 by controlling a flow rate of the carrier gas toward the sample injection mechanism 31 (TOC measurement state). Further, the flow controller 5 temporarily supplies carrier gas from the gas source 4 to the syringe 1 via the pipe 51 by controlling a flow rate of the carrier gas to the syringe 1 side (IC removal state).

After a sample is sucked into the syringe 1 from the sample storage unit 21B, acid is added into the syringe 1 from the acid storage unit 22B, and the flow controller 5 is brought into the IC removal state. Then, liquid (mixed liquid of the sample and the acid) in the syringe 1 is aerated by carrier gas supplied into the syringe 1. In this manner, IC contained in the sample in the syringe 1 can be converted into carbon dioxide and removed. At this time, carbon dioxide generated in the syringe 1 is released into the atmosphere through the pipe 25A.

After the above, in a state where carrier gas is supplied from the flow controller 5 to the sample injection mechanism 31 side via the pipe 41 (TOC measurement state), the sample from which IC is removed is injected from the syringe 1 into the reaction tube 32 via the sample injection mechanism 31. In this way, the sample from which IC is removed is caused to react in the reaction tube 32, and carbon dioxide generated in the reaction tube 32 is detected by the detection unit 35, so that TOC can be measured.

2. Specific configuration of humidifier

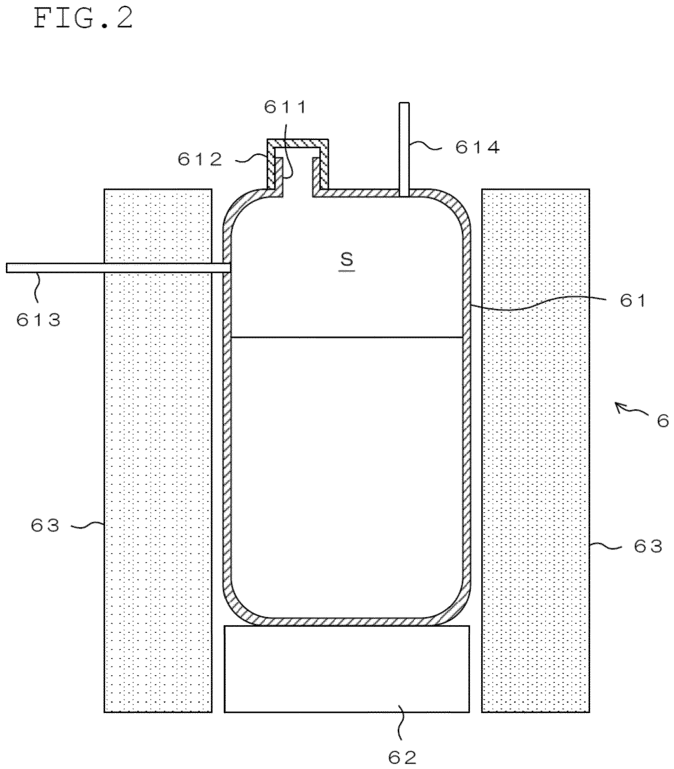

FIG. 2 is a schematic cross-sectional view illustrating a configuration example of the humidifier 6. The humidifier 6 is of a steam type, and generates water vapor by heating water. The humidifier 6 includes a water storage unit 61, a heater 62, a heat insulating material 63, and the like.

The water storage unit 61 is a hollow member, and can store water (for example, pure water) in the inside. The water storage unit 61 may be formed of a material having high thermal conductivity such as metal, for example. A water supply port 611 for supplying water into the water storage unit 61 is formed in an upper portion of the water storage unit 61. A cap 612 is detachably attached to the water supply port 611. Water in the water storage unit 61 decreases as the humidifier 6 is used. Therefore, the user periodically performs work of detaching the cap 612 to open the water supply port 611, supplying water from the water supply port 611 into the water storage unit 61, and then attaching the cap 612 to the water supply port 611.

The heater 62 constitutes a water heating unit for heating water in the water storage unit 61 by heating the water storage unit 61 from the outside. In this manner, temperature of the water in the water storage unit 61 is adjusted to, for example, about 45°C. For example, the heater 62 heats a bottom surface of the water storage unit 61. However, the heater 62 may be configured to heat a surface (for example, a side surface) other than the bottom surface of the water storage unit 61.

A side surface of the water storage unit 61 is enclosed by the heat insulating material 63, and the heat insulating material 63 can prevent heat of water in the water storage unit 61 from escaping to the outside. A material of the heat insulating material 63 is not particularly limited, but for example, urethane foam or the like is used. Note that the heat insulating material 63 may be omitted.

Water is stored in the water storage unit 61 such that a space S is formed in an upper portion of the water storage unit 61. Therefore, the space S is filled with water vapor generated as water in the water storage unit 61 is heated by the heater 62. A gas inflow pipe 613 and a gas outflow pipe 614 communicate with the space S in the water storage unit 61. Positions at which the gas inflow pipe 613 and the gas outflow pipe 614 communicate with the space S are not particularly limited, but as illustrated in FIG. 2, the gas outflow pipe 614 may communicate with the space S above the gas inflow pipe 613.

The gas inflow pipe 613 and the gas outflow pipe 614 constitute a part of the pipe 41. That is, carrier gas from the gas source 4 flows into the space S in the water storage unit 61 via the gas inflow pipe 613, then flows out of the water storage unit 61 from the gas outflow pipe 614 through the space S, and is introduced into the reaction tube 32 via the sample injection mechanism 31.

Water vapor filling the space S is mixed with carrier gas in a process of passing through the space S in the water storage unit 61. In this manner, carrier gas introduced into the reaction tube 32 becomes inert gas containing water vapor. Since water vapor generated from the steam type humidifier 6 does not contain impurities, it is possible to prevent generation of detection noise due to introduction of TOC contained in water in the water storage unit 61 into the reaction tube 32.

By use of the humidifier 6 as described above, water vapor can be continuously and stably mixed with carrier gas introduced into the reaction tube 32. In this manner, an amount of water vapor in the carrier gas introduced into the reaction tube 32 can be maintained at a predetermined value or more. Humidity of the carrier gas introduced into the reaction tube 32 may be 10 to 80 g/m$^3$. Note that, in a case where a sample is water, water vapor is generated only by introduction of the sample into the reaction tube 32. However, since the water vapor generated from the sample quickly flows out of the reaction tube 32 due to carrier gas, it is difficult to stably secure a necessary amount of water vapor over general oxidation reaction time (for example, about two to five minutes).

3. Generation principle of carbon dioxide

In the present embodiment, organic carbon in a sample undergoes steam reforming reaction so that carbon dioxide is generated. Specifically, in a case where inert gas containing water vapor is introduced into the reaction tube 32 as carrier gas, reaction represented by Equation (1) below occurs in the reaction tube 32 having an oxidation catalyst. At this time, the reaction tube 32 is heated to, for example, 500 to 1100°C. In Equation (1) below, $C_mH_n$ is TOC (total organic carbon) contained in a sample.
[Equation 1]

$$C_mH_n + mH_2O \rightleftarrows mCO + \left(m + \frac{n}{2}\right)H_2 \qquad \cdots (1)$$

Since the carrier gas contains water vapor ($H_2O$), TOC contained in the sample reacts with water vapor in the reaction tube 32. This reaction does not require oxygen. Further, carbon dioxide ($CO_2$) and hydrogen ($H_2$) are finally obtained by generation of reaction (aqueous gas shift reaction) represented by Equation (2) in association with the reaction represented by Equation (1) above.
[Equation 2]

$$CO + H_2O \rightleftharpoons CO_2 + H_2 \qquad \cdots (2)$$

In Equation (1), a mole ratio of water vapor to carbon is called a steam/carbon ratio (S/C ratio), and in a case where this value is smaller than a stoichiometric ratio, reaction is not completed, and TOC remains on an oxidation catalyst in the reaction tube 32. Therefore, when water vapor having an S/C ratio or more is contained in carrier gas, the whole TOC in a sample react.

For example, assuming that a flow rate of carrier gas is 150 mL/min, in a case where 100 pL of a sample containing 50 mg/L of TOC is introduced into the reaction tube 32, an amount of carbon introduced into the reaction tube 32 is 0.005 mg, that is, 0.005/12 mmol. A water vapor amount equivalent to the carbon amount is 0.005 / 12 × 18 = 0.0075 mg. This amount of water vapor needs to be always present in the carrier gas introduced into the reaction tube 32 at 150 mL/min. For this purpose, an amount of water vapor in the carrier gas only needs to be 0.0075 / 150 × 1000$^3$ = 50 g/m$^3$ or more. This water vapor amount corresponds to a saturated water vapor amount at an air temperature of about 40°C.

Note that hydrogen generated by the reactions of Equations (1) and (2) described above is about several ppm and is an extremely small amount, and thus there is no risk of explosion. Therefore, the generated hydrogen may be discharged without further processing, or a processing unit for performing specific processing on the generated hydrogen may be provided.

4. Specific example of total organic carbon measurement method

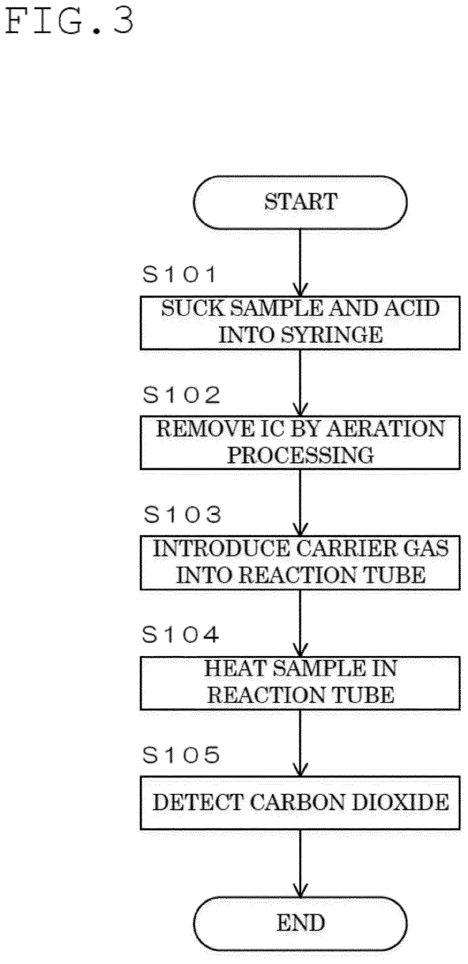

FIG. 3 is a flowchart for explaining a method for measuring total organic carbon. In a case of measuring TOC contained in a sample, first, the sample is sucked into the syringe 1, and after that, acid is sucked into the syringe 1 (Step S101: acid addition step). An amount of the sample sucked into the syringe 1 is, for example, 500 μL. On the other hand, an amount of acid sucked into the syringe 1 is smaller than an amount of the sample, and is, for example, 10 μL.

After the above, when carrier gas is supplied into the syringe 1, liquid (mixed liquid of the sample and the acid) in the syringe 1 is aerated for about 90 seconds. In this manner, IC contained in the sample in the syringe 1 is removed (Step S102: aeration step).

The sample from which IC is removed in this manner is supplied from the syringe 1 to the reaction tube 32. A supply amount of the sample to the reaction tube 32 is, for example, 100 pL. At this time, carrier gas containing water vapor is introduced into the reaction tube 32 from the gas source 4 via the humidifier 6 (Step S103: carrier gas introduction step). Note that the carrier gas is not introduced into the reaction tube 32 for the first time at this time, but is continuously supplied to the detection unit 35 via the reaction tube 32 from when the device is turned on to when the device is turned off.

As a result, the sample is heated in the reaction tube 32, and TOC contained in the sample reacts with water vapor, so that carbon dioxide is generated from the sample (Step S104: sample heating step). Carbon dioxide generated from the sample is guided to the detection unit 35 by the carrier gas and detected by the detection unit 35 (Step S105: detection step).

5. Variation

In the above embodiment, the configuration in which the heater 62 indirectly heats water in the water storage unit 61 by heating the water storage unit 61 from the outside is described. However, the present invention is not limited to such a configuration. For example, a configuration in which the heater 62 is provided in the water storage unit 61 to directly heat water in the water storage unit 61 may be employed.

The humidifier 6 is not limited to one of a steam type, and may have another configuration such as an ultrasonic type. It is also possible to downsize the humidifier 6 and downsize the entire device depending on a system to be employed. In a humidifier of an ultrasonic type, water is atomized as vibration is applied to water using an ultrasonic wave so that water vapor is generated. However, in a case of the configuration in which water is atomized and mixed with carrier gas, detection noise may occur due to TOC contained in the water being introduced into the reaction tube 32, and thus, it may be difficult to employ the configuration depending on a condition such as a type of a sample.

The sample is not limited to liquid, and may be a solid. Even in such a case, inert gas containing water vapor only needs to be introduced as carrier gas into a reaction tube in which a solid sample is heated to generate carbon dioxide.

In the above embodiment, the configuration in which IC is converted into carbon dioxide and removed by adding acid to a sample and aerating the sample, and carbon dioxide generated when the sample from which IC is removed caused to react in the reaction tube 32 is detected by the detection unit 35. However, the present invention is also applicable to a configuration in which TC and IC are measured, and a difference between them (TC-IC) is calculated as TOC. In this case, when TC is measured, carrier gas containing water vapor may be introduced into the reaction tube together with the sample.

6. Aspect

It is to be understood by those skilled in the art that a plurality of the exemplary embodiments described above are specific examples of an aspect described below.

(Item 1) A total organic carbon measurement device according to one aspect may include:

a sample heating unit that has a space in which an oxidation catalyst is arranged and heats a sample arranged in the space;
a carrier gas introduction unit that introduces inert gas containing water vapor as carrier gas into the sample heating unit; and
a detection unit for detecting carbon dioxide generated by steam reforming reaction of organic carbon in a sample in the sample heating unit.

According to the total organic carbon measurement device described in Item 1, carbon dioxide can be generated from total organic carbon in a sample by an action of water vapor contained in carrier gas, and therefore inert gas can be used as the carrier gas.

(Item 2) In the total organic carbon measurement device according to Item 1, the carrier gas introduction unit may include a water vapor generation unit that generates water vapor, and cause carrier gas mixed with water vapor generated in the water vapor generation unit to be introduced into the sample heating unit.

According to the total organic carbon measurement device described in Item 2, carrier gas containing water vapor can be introduced into the sample heating unit only by mixing of water vapor generated in the water vapor generation unit with the carrier gas.

(Item 3) In the total organic carbon measurement device according to Item 2, the carrier gas introduction unit may include a pipe that allows the water vapor generation unit and the sample heating unit to communicate with each other, and a pipe heating unit that heats the pipe.

According to the total organic carbon measurement device described in Item 3, since carrier gas mixed with water vapor can be introduced into the sample heating unit while being heated, it is possible to prevent water vapor in the carrier gas from being reduced in amount before being introduced into the sample heating unit.

(Item 4) In the total organic carbon measurement device according to Item 2 or 3, the water vapor generation unit may include a water storage unit for storing water and a water heating unit for heating water in the water storage unit, and water vapor generated as water in the water storage unit is heated may be mixed with carrier gas passing through the water storage unit.

According to the total organic carbon measurement device described in Item 4, it is possible to generate water vapor with a simple configuration of only heating water in the water storage unit, mix the water vapor with carrier gas, and introduce the mixture into the sample heating unit.

(Item 5) A total organic carbon measurement method according to one aspect may include:

a carrier gas introduction step of introducing inert gas containing water vapor as carrier gas into a sample heating unit having a space in which an oxidation catalyst is arranged;
a sample heating step of heating a sample arranged in the space in the sample heating unit and causing organic carbon in the sample to undergo steam reforming reaction to generate carbon dioxide; and
a detection step of detecting the carbon dioxide that is generated.

According to the total organic carbon measurement method described in Item 5, carbon dioxide can be generated from total organic carbon in a sample by an action of water vapor contained in carrier gas, and therefore inert gas can be used as the carrier gas.

(Item 6) In the total organic carbon measurement method according to Item 5, in the sample heating step, carbon dioxide may be generated based on reaction of total organic carbon contained in a sample with water vapor.

According to the total organic carbon measurement method described in Item 6, total organic carbon can be measured by detection of carbon dioxide generated based on reaction of total organic carbon contained in a sample with water vapor.

(Item 7) In the total organic carbon measurement method according to Item 5 or 6, in the carrier gas introduction step, water vapor may be generated in a water vapor generation unit, and carrier gas mixed with water vapor generated in the water vapor generation unit may be introduced into the sample heating unit.

According to the total organic carbon measurement method described in Item 7, carrier gas containing water vapor can be introduced into the sample heating unit only by mixing of water vapor generated in the water vapor generation unit with the carrier gas.

(Item 8) In the total organic carbon measurement method according to Item 7, in the carrier gas introduction step, while a pipe that causes the water vapor generation unit and the sample heating unit to communicate with each other is heated, carrier gas mixed with water vapor may be introduced into the sample heating unit through the pipe.

According to the total organic carbon measurement method described in Item 8, since carrier gas mixed with water vapor can be introduced into the sample heating unit while being heated, it is possible to prevent water vapor in the carrier gas from being reduced in amount before being introduced into the sample heating unit.

(Item 9) In the total organic carbon measurement method according to Item 7 or 8, the water vapor generation unit may include a water storage unit for storing water and a water heating unit for heating water in the water storage unit, and

in the carrier gas introduction step, water vapor generated as water in the water storage unit is heated may be mixed with carrier gas passing through the water storage unit.

According to the total organic carbon measurement method described in Item 9, it is possible to generate water vapor with a simple configuration of only heating water in the water storage unit, mix the water vapor with carrier gas, and introduce the mixture into the sample heating unit.

DESCRIPTION OF REFERENCE SIGNS

| 1 | syringe |
|---|---|
| 2 | flow path switching unit |
| 3 | TOC measurement unit |
| 4 | gas source |
| 5 | flow controller |
| 6 | humidifier |
| 7 | control unit |
| 8 | display unit |
| 21 | sample supply unit |
| 22 | addition unit |
| 30 | sample heating unit |
| 31 | sample injection mechanism |
| 32 | reaction tube |
| 33 | electric furnace |
| 34 | dehumidifier |
| 35 | detection unit |
| 40 | carrier gas introduction unit |
| 41 | pipe |
| 41a | pipe heating unit |
| 61 | water storage unit |
| 62 | heater |
| 63 | heat insulating material |

**Claims**

1. A total organic carbon measurement device comprising:

   a sample heating unit that has a space in which an oxidation catalyst is arranged and heats a sample arranged in the space;
   a carrier gas introduction unit that introduces inert gas containing water vapor as carrier gas into the sample heating unit; and
   a detection unit for detecting carbon dioxide generated by steam reforming reaction of organic carbon in a sample in the sample heating unit.

2. The total organic carbon measurement device according to claim 1, wherein
   the carrier gas introduction unit includes a water vapor generation unit that generates water vapor, and causes carrier gas mixed with water vapor generated in the water vapor generation unit to be introduced into the sample heating unit.

3. The total organic carbon measurement device according to claim 2, wherein
the carrier gas introduction unit includes a pipe that allows the water vapor generation unit and the sample heating unit to communicate with each other, and a pipe heating unit that heats the pipe.

4. The total organic carbon measurement device according to claim 2, wherein
the water vapor generation unit includes a water storage unit for storing water and a water heating unit for heating water in the water storage unit, and water vapor generated as water in the water storage unit is heated is mixed with carrier gas passing through the water storage unit.

5. A total organic carbon measurement method comprising:

a carrier gas introduction step of introducing inert gas containing water vapor as carrier gas into a sample heating unit having a space in which an oxidation catalyst is arranged;
a sample heating step of heating a sample arranged in the space in the sample heating unit and causing organic carbon in the sample to undergo steam reforming reaction to generate carbon dioxide; and
a detection step of detecting the carbon dioxide that is generated.

6. The total organic carbon measurement method according to claim 5, wherein
in the sample heating step, carbon dioxide is generated based on reaction of total organic carbon contained in a sample with water vapor.

7. The total organic carbon measurement method according to claim 5, wherein
in the carrier gas introduction step, water vapor is generated in a water vapor generation unit, and carrier gas mixed with water vapor generated in the water vapor generation unit is introduced into the sample heating unit.

8. The total organic carbon measurement method according to claim 7, wherein
in the carrier gas introduction step, while a pipe that causes the water vapor generation unit and the sample heating unit to communicate with each other is heated, carrier gas mixed with water vapor is introduced into the sample heating unit through the pipe.

9. The total organic carbon measurement method according to claim 7, wherein

the water vapor generation unit includes a water storage unit for storing water and a water heating unit for heating water in the water storage unit, and
in the carrier gas introduction step, water vapor generated as water in the water storage unit is heated is mixed with carrier gas passing through the water storage unit.

EP 4 123 302 A1

FIG.1

22A (22)

22B (22)
ACID

21A (21)

24A

21B (21)
SAMPLE

51

5 (40)

12

25A

2

1

11

23A

(40) 41

(40) 31

3

32 (30)
322
33 (30)
321

34
DEHUMIDIFIER

35
DETECTION UNIT

41A (40)

4 (40)
GAS SOURCE

FLOW CONTROLLER

HUMIDIFIER

6 (40)

7
CONTROL UNIT

8
DISPLAY UNIT

10

FIG.2

# FIG.3

```
          ╭─────────────╮
          │    START     │
          ╰──────┬──────╯
                 │
S101             ▼
   ┌─────────────────────────┐
   │  SUCK SAMPLE AND ACID   │
   │      INTO SYRINGE       │
   └─────────────┬───────────┘
                 │
S102             ▼
   ┌─────────────────────────┐
   │  REMOVE IC BY AERATION  │
   │       PROCESSING        │
   └─────────────┬───────────┘
                 │
S103             ▼
   ┌─────────────────────────┐
   │  INTRODUCE CARRIER GAS  │
   │    INTO REACTION TUBE   │
   └─────────────┬───────────┘
                 │
S104             ▼
   ┌─────────────────────────┐
   │     HEAT SAMPLE IN      │
   │      REACTION TUBE      │
   └─────────────┬───────────┘
                 │
S105             ▼
   ┌─────────────────────────┐
   │  DETECT CARBON DIOXIDE  │
   └─────────────┬───────────┘
                 │
                 ▼
          ╭─────────────╮
          │     END      │
          ╰─────────────╯
```

## INTERNATIONAL SEARCH REPORT

| | |
|---|---|
| International application No. | |
| | PCT/JP2021/007834 |

**A. CLASSIFICATION OF SUBJECT MATTER**
Int. Cl. G01N31/00(2006.01)i, G01N31/10(2006.01)i
FI: G01N31/00 D, G01N31/10

According to International Patent Classification (IPC) or to both national classification and IPC

**B. FIELDS SEARCHED**

Minimum documentation searched (classification system followed by classification symbols)
Int. Cl. G01N31/00, G01N31/10

Documentation searched other than minimum documentation to the extent that such documents are included in the fields searched
Published examined utility model applications of Japan    1922-1996
Published unexamined utility model applications of Japan   1971-2021
Registered utility model specifications of Japan       1996-2021
Published registered utility model applications of Japan   1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 62-190465 A (SHIMADZU CORP.) 20 August 1987, claims | 1-9 |
| A | JP 51-105886 A (SUMITOMO CHEMICAL CO., LTD.) 20 September 1976, examples | 1-9 |
| A | Microfilm of the specification and drawings annexed to the request of Japanese Utility Model Application No. 146275/1974 (Laid-open No. 73088/1976) (TORAY INDUSTRIES, INC.) 09 June 1976, claims | 1-9 |
| A | JP 52-48039 B2 (KABUSHIKI KAISHA YOKOGAWA DENKI SEISAKUJO) 07 December 1977, claims | 1-9 |

☒ Further documents are listed in the continuation of Box C.    ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23.04.2021 | 25.05.2021 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
|---|
| PCT/JP2021/007834 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-43186 A (IDEMITSU KOSAN CO., LTD.) 17 February 2005, paragraph [0016] | 1-9 |
| A | JP 6-45882 Y2 (TOKICO, LTD.) 24 November 1994, examples | 1-9 |
| A | JP 10-160720 A (TOKICO, LTD.) 19 June 1998, paragraphs [0009], [0010] | 1-9 |
| A | JP 2000-155117 A (SHIMADZU CORP.) 06 June 2000, claim 1 | 1-9 |
| A | JP 2011-21938 A (HONDA MOTOR CO., LTD.) 03 February 2011, paragraphs [0030]-[0032] | 1-9 |
| A | WO 2009/153264 A1 (COMMISSARIAT A L'ENERCIE ATOMIQUE) 23 December 2009, p. 33 | 1-9 |

Form PCT/ISA/210 (continuation of second sheet) (January 2015)

**INTERNATIONAL SEARCH REPORT**
Information on patent family members

International application No.

PCT/JP2021/007834

| Patent Documents referred to in the Report | Publication Date | Patent Family | Publication Date |
|---|---|---|---|
| JP 62-190465 A | 20.08.1987 | (Family: none) | |
| JP 51-105886 A | 20.09.1976 | (Family: none) | |
| JP 51-73088 U1 | 09.06.1976 | (Family: none) | |
| JP 52-48039 B2 | 07.12.1977 | (Family: none) | |
| JP 2005-43186 A | 17.02.2005 | (Family: none) | |
| JP 6-45882 Y2 | 24.11.1994 | (Family: none) | |
| JP 10-160720 A | 19.06.1998 | (Family: none) | |
| JP 2000-155117 A | 06.06.2000 | (Family: none) | |
| JP 2011-21938 A | 03.02.2011 | (Family: none) | |
| WO 2009/153264 A1 | 23.12.2009 | FR 2932888 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007093209 A